# EUROPEAN PATENT SPECIFICATION

(11) **EP 2 117 514 B1**
(45) Date of publication and mention of the grant of the patent: **15.06.2011**
(21) Application number: 08720138.0
(22) Date of filing: 29.02.2008
(51) Int. Cl.: A61K 9/19, A61K 9/08, A61K 47/10, A61K 47/12, A61K 47/18, A61K 47/26, A61K 9/00, A61K 38/00, A61K 47/48, A61K 38/21

(54) **COMPOSITIONS COMPRISING PEG- INTERFERON ALPHA CONJUGATES AND RAFFINOSE AS CRYOPROTECTANT**
ZUSAMMENSETZUNGEN MIT PEG-INTERFERON-ALPHA-KONJUGATEN UND RAFFINOSE ALS KRYOSCHUTZMITTEL
COMPOSITIONS COMPRENANT DES CONJUGUÉS DE PEG-INTERFÉRON ALPHA ET DE LA RAFFINOSE EN TANT QUE CRYOPROTECTEUR

(30) Priority: 05.03.2007 IN MU04122007
(43) Date of publication of application: 18.11.2009
(73) Proprietor: Cadila Healthcare Limited, Ahmedabad 380 015, Gujarat (IN)
(72) Inventor: BANDYOPADHYAY, Sanjay, Ahmedabad 380 015 Gujarat (IN); MENDIRATTA, Sanjeev, Kumar, Ahmedabad 380 015 Gujarat (IN); NATARAJAN, Venkatesan, Ahmedabad 380 015 Gujarat (IN); PATEL, Pankaj, Ramanbhai, Ahmedabad 380 015 (IN)
(74) Representative: Srinivasan, Ravi Chandran
(86) International application number: PCT/IN2008/000114
(87) International publication number: WO 2008/107908

(56) References cited:
- US-A1- 2005 031 576
- US-B1- 6 180 096

## Description

### Field of invention

The present invention relates to novel lyophilized and stabilized formulations of PEG-interferon alpha conjugates and processes for their preparation.

### Background of the invention

The major disadvantage with the therapeutic use of most biologicals is that they are administered through parenteral route e.g., intravenously (i.v.), subcutaneously (s.c.), intramuscularly (i.m.) etc., which means that delivery to the patient is associated with pain and discomfort. Further, because of their usually very short half-lives, biologicals require frequent administrations into the patient in order to maintain therapeutic blood levels of the drug. Many types of injections that cannot be self-administered require frequent trips to the clinic, further adding to the discomfort of the patient. Multiple examples of such biological drugs that require frequent administration exist. Interferon alpha -2a (Roferon, Roche) and interferon alpha-2b (Intron A, Schering AG), the two recombinant forms of human interferon alpha, used in the treatment of chronic hepatitis B and C have a serum half-life of less than 12 h (McHutchison, et al., Engl. J. Med. 1998, 339, 1485-1492; Glue, et al., Clin. Pharmacol. Ther. 2000, 68, 556-567) and therefore requiring at least 3 times a week administration of the drug. Repeated injections with interferon beta-1b (Betaseron) are also required to treat the patients of multiple sclerosis (MS). The recommended dosing is by subcutaneous route given every other day. Another example of a drug where repeated injections are required is filgrastim (granulocyte colony stimulating factor, or G-CSF), where the injection is given everyday for the duration of treatment of two weeks.

One very successful and well accepted method of overcoming the above drawbacks of frequent high dose injections to maintain threshold levels of the drug in the body is to increase the in-vivo half-life of the therapeutic protein by conjugating it with a polymer, preferably polyethylene glycol (PEG). It is believed that PEG molecules with their long chains create a protective shield around the pegylated drug molecule in aqueous solution, and thereby, help in reducing the immunogenicity of protein drugs while also protecting them from the action of proteases. In addition to this, because of their large hydrodynamic volume, PEG molecules are able to reduce the rate of kidney clearance of the drug while extending the half-life of the protein drug in circulation. Conjugation of proteins to PEG has been reported since 1970s. Usually PEG moieties are attached to the protein by first activating the PEG moiety and then reacting it with the side chains of lysine residues and/or the N-terminal amino group on the protein. The most frequently used PEG is monofunctional PEG because this moiety resists cross-linking and aggregation. One such example has been disclosed by Davis et al. in US Pat. No. 4,179,337. PEG-protein conjugates were formed by reacting a biologically active material with a molar excess concentration of a highly activated polymer having a terminal linking group without regard to where the polymer would attach to the protein, and leading to a physiologically active non-immunogenic water soluble polypeptide composition. Pegylation of interferons has been reported in U.S. Pat. Nos. 4,766,106 and 4,917,888 which describe inter alia beta interferon conjugated with activated polymers including mPEG-2,4,6-trichloro-S-triazine, mPEG-N-succinimidyl glutarate or mPEG-N-succinimidyl succinate. One such disclosure in U.S. Pat. No. 5,951,974 describes the conjugation of interferon to a substantially non-antigenic polymer at a histidine site. Another such disclosure in U.S. Pat. No. 5,981,709 describes the alpha interferon-polymer conjugate with relatively long circulating half-life *in-vivo.*

Some commercially, available pegylated therapeutic proteins include, ADAGEN (pegylated bovine adenosine deaminase), which is used to treat X-linked severe combined immunodeficiency syndrome; PEGASYS (pegylated alpha-interferon 2a), which is used in the treatment of hepatitis C; PEG-Intron (pegylated alpha-interferon 2b) for chronic hepatitis C; Oncaspar (pegylated L-asparaginase) for the treatment of acute lymphoblastic leukemia in patients who are hypersensitive to the native unmodified form of L-asparaginase; and, Neulasta (pegylated recombinant methionyl human granulocyte colony stimulating factor) for cancer chemotherapy induced neutropema.

Hepatitis C virus (HCV) is one of the major causes of liver disease in the world. Nearly 200 million people are affected world wide. Interferon in combination with Ribavirin has been shown to be effective in decreasing the viral load of patients with chronic hepatitis C, however it needs to be given three times a week. PEG-interferon alpha 2b is a covalent conjugate of recombinant interferon alpha 2b with monomethoxy PEG in a 1:1 molar ratio (Glue P et al., Clin Pharmacol Ther. 2000; 68; 556-567). The mean absorption half-life of PEG-interferon alpha 2b is 5 fold greater than non-pegylated interferon alpha-2b. The mean elimination half-life is 40 hours in patients with hepatitis C infection. Another product is PEG-interferon alpha 2a, which has a 40kDa branched chain molecule with each PEG branch with an average molecular weight of 20kDa. The two monomethoxy PEG chains are joined via hydrolytically stable urethane bonds to a lysine linker molecule, one at the lysine alpha-amino group and another at the lysine ε-amino group. The mean absorption half-life of PEG-interferon alpha 2a is 10 fold greater than non-pegylated interferon alpha-2a. The mean elimination half-life is about 60 hours in patients with hepatitis C infection. Both these improved products need to be administered at once a week regimen only.

While, some protein-polymer conjugates are stable in the liquid form, others are not. For example, unlike the case of PEG-interferon alpha 2a where pegylation leads to a stable urethane bond, which is primarily stable in aqueous media, the PEG-interferon alpha 2b product, which contains PEG primarily linked to a histidine (His 34) residue, is highly unstable in the liquid form. With such protein-polymer conjugates, one has to use techniques such as lyophilization/freeze-drying - a process whereby water is sublimed from a composition after it is frozen - which can provide a stable form to the biological over a desired period of time. Thus, to make a stable formulation of PEG-interferon alpha 2b, one needs to carefully lyophilize the formulation with suitable cryoprotectant(s) or lyoprotectant(s), and stabilizers, that stabilize the pegylated interferon alpha conjugates to prevent depegylation during and after lyophilization - a phenomenon commonly associated with the PEG-interferon alpha 2b product. Further, besides the cryoprotectant and stabilizers the lyophilized formulation also contains bulking agents to increase the amount of the solid material in the vial.

One specific way, in which the problem of instability of urethane linkage at His 34 residue has been resolved in the case of PEG-interferon alpha 2b, is by utilizing a formulation that has been disclosed in U.S. Pat. 6,180,096, where in the PEG-IFN alpha 2b conjugates are lyophilized in the presence of buffer, cryoprotectants, a stabilizer and a solvent of which one such formulation contains a disaccharide sucrose, as a cryoprotectant, along with, monobasic sodium phosphate dihydrate and dibasic sodium phosphate anhydrous as buffer, with polysorbate 80 as a stabilizer, and water as a solvent. While the above formulation is commercially successful in the treatment of Hepatitis C, it is nevertheless associated with several problems some of which are elaborated in another patent application, WO2006/020720, by the same company, that sites longer lyophilization cycles leading to increased cost of manufacturing, and higher moisture content associated with the commercial formulation, as some of the reasons to discover and report novel formulation in WO2006/020720. In WO2006/020720, the inventors disclose another lyophilized formulation of PEG-IFN alpha 2b, wherein the cryoprotectant comprises of at least 60% trehalose, the buffering components comprise of monobasic sodium phosphate dihydrate and dibasic sodium phosphate anhydrous, and where the formulation further comprises of polysorbate 80 as a stabilizer and water as a solvent, and that is able to overcome the above described problems of the commercial formulation. The need for additional formulations for the protection of PEG-IFN alpha-2b conjugates cannot be better emphasized than the fact that the assignees of US patent 6,180,096 (commercial formulation), and the applicants of WO2006/020720, are the same company, Schering Corporation, that is continuing to develop and disclose more lyophilized formulations for PEG-IFN alpha-2b.

With US2005031576 the production of pegylated PEG-interferon alpha is explained in Example 13. Possible pharmaceutical compositions are anticipated by the suggestion to include carbohydrates, buffer, antioxidants, surfactants, a solvent.

The need for additional formulations of PEG-IFN alpha-2b to those in existence is with an aim not only to protect the PEG-interferon alpha conjugate during and after lyophilization, but also to have a long-term storage when lyophilized in an appropriate container. The process of such formulations should be easy to handle and be more cost-effective than those used for the current formulation. The current commercial formulation of PEG-IFN alpha-2b, which is sucrose based (as described in US patent 6,180,096), has a rather long lyophilization cycle, as described in PCT/US2005/028441. The formulation disclosed in the current invention uses a lyophilization cycle, where the process time is reduced at least by 48 h with respect to some of the other formulations known. This, in turn, will help in bringing down the cost of manufacturing of this drug, significantly.

The present invention provides novel lyophilized and stabilized formulations of PEG-interferon alpha conjugates and the process for their preparation.

### Embodiments of the Invention

In one embodiment of this invention is provided novel lyophilized formulations of PEG-interferon alpha conjugates.

In another embodiment of the invention is provided a process for the preparation of novel formulations of PEG-interferon alpha conjugates.

### Description of the Invention

The present invention relates to novel lyophilized and stabilized formulations of PEG-interferon alpha conjugates and process(es) for their preparation. The formulations involve formulating PEG-interferon alpha conjugates with suitable buffer(s), suitable cryoprotectant(s), suitable stabilizer(s) and a solvent, optionally with other suitable excipients, which is subsequently lyophilized.

It will be appreciated that the present invention is not limited by the concentrations of the components in the novel formulations as is disclosed in the specification.

PEG-interferon alpha conjugates according to the present invention are Interferon alpha molecules or their variants covalently linked to one or more PEG molecule/s. Suitable PEG molecules may be those well known and used in preparing pegylated biological products for therapeutic use. The PEG-interferon alpha conjugates of the present invention may comprise of Interferon alpha-2a, Interferon alpha-2b or Interferon alpha-2c and their suitable variants, most preferably, the PEG-interferon alpha-2b conjugate.

Polymers are molecules comprising covalently linked repeating chemical units. Often, the approximate molecular weight of the polymer is designated with a number following the name of the repeated chemical unit. For example, "PEG₁₂₀₀₀" or "polyethylene glycol (12000)" refers to a polymer of polyethylene glycol having an average molecular weight of approximately 12,000 daltons.

Conjugation of polymers to proteins may result in a single polymer molecule conjugated to a protein or multiple such conjugations to a single protein. The degree of conjugation and site of pegylation are mainly dependent upon the reaction conditions and PEG / protein ratio. In a preferred embodiment, the PEG-interferon alpha conjugate in the formulations of the present invention comprises a single interferon alpha-2b conjugated to a single PEG molecule, predominantly. In a still preferred embodiment, the PEG-interferon alpha conjugate in the formulations of the present invention comprises a single interferon alpha-2b conjugated to a single PEG₁₂₀₀₀ molecule. In a particularly preferred embodiment, the Interferon alpha-2b molecule is linked to the PEG₁₂₀₀₀ molecule with a urethane bond. Several processes for preparing PEG-IFN conjugates are known in the art and such processes and the products derived from such processes are considered to be encompassed within the scope of the present invention. Examples of such process(es) for producing this protein-polymer conjugate may be found in U.S. Pat. No. 5612460 (Zalipsky) and U.S. Pat. No. 5711944 (Gilbert et al). Without limiting the scope of the present invention, when such a protein-polymer conjugate is utilized in the formulation solutions of the present invention, the preferred concentration of PEG-interferon alpha conjugate is 0.03 to 2.0 mg interferon alpha per ml. When a single interferon alpha molecule is linked to a single PEG₁₂₀₀₀ molecule, the resulting conjugated PEG-interferon alpha conjugates may be in the form of a single or mixture of different positional isomers.

To preserve the PEG-interferon alpha conjugate in the most stable and active form, lyophilization may be used. Lyophilization is a process of freeze-drying a composition wherein a frozen aqueous mixture is treated to remove water. Commonly, the process involves the sublimation of water from the aqueous solutions, usually under reduced pressure conditions. After lyophilization, the PEG-interferon alpha conjugate(s) can be stored for extended periods of time.

PEG-interferon alpha conjugates, however, are subject to damage during and after lyophilization (US 6,180,096). Hence, there is a need to suitably formulate the PEG-interferon alpha conjugates so as to protect them from degradation during and after lyophilization. Moreover, it will also be useful if such formulations provide physical strength to the formulation.

The present invention protects PEG-interferon alpha conjugates from damage by including them in formulations that prevent damage during and after lyophilization. While the present invention is not limited to a particular formulation, the formulations that are anticipated here utilize a suitable buffer(s), suitable stabilizer(s), suitable cryoprotectant(s) and/or lyoprotectant(s), a bulking agent(s) and solvent(s), alone or in suitable combination, optionally with other suitable excipients, in addition to the PEG-interferon alpha conjugate. Various possible combinations of the selected groups of buffers, stabilizers and cryoprotectants as described below, may be used to prepare the novel formulations of the present invention.

Buffers are suitable for maintaining pH of the formulation. The buffer system, which may be used comprises of phosphate, succinate, histidine, glycinate and the like, either alone or in suitable combination, which provides the desired pH range. The preferred pH range is between 4.5-7.1, preferably 6.5-7.1 and most preferably 6.8. The preferred molar concentration of the buffer is in the range of 0.001 to 0.5 M. Preferred buffers may be selected from sodium phosphate, sodium succinate, potassium succinate, histidine chloride, sodium glycinate or their suitable combinations. Other buffer systems may also be used which maintain the desired pH range.

The term "cryoprotectants" generally includes agents, which provide stability to the protein from freezing-induced stresses; however, the term also includes agents that provide stability, e.g., to bulk drug formulations during storage from non-freezing-induced stresses. Examples of cryoprotectants include different polyols and saccharides e.g. sucrose, lactose, trehalose, raffinose and mannitol, or their suitable combinations.

The term "cryoprotectant" includes agents that provide stability to the protein during water removal from the system during the drying process, presumably by maintaining the proper conformation of the protein through hydrogen bonding. Cryoprotectants can also have lyoprotectant effects; therefore, the terms "cryoprotectant" and "lyoprotectant" are used interchangeably, herein.

A stabilizing agent is useful in the prevention of adsorption of the PEG-interferon alpha conjugates to glass and stainless steel surfaces of the process equipments used to make and store the formulation. Suitable stabilizing agents, which may be used are sodium dodecyl sulphate (SDS), polysorbates (e.g., Polysorbate 20, 40 or 80, either alone, or in combination). Examples may be from the class of poly(oxy-1,2-ethanediyl) derivatives. One such preferred stabilizing agent is Polyoxyethylene 20 Sorbitan Mono-oleate, polysorbate 80 (Tween 80) at a preferred concentration of 0.01 to 1 mg / mL.

The present invention is not limited to any specific amount of cryoprotectant. The cryoprotectant may be used alone or in suitable combinations. In one embodiment, cryoprotectants are present in an amount of 0.05% to 90%, preferably 0.05 to 50% and most preferably in an amount of 0.15 to 20% based on the total weight of PEG-interferon solution.

Suitable solvent for the present formulation is water, preferably the solvent may be water for injection.

Other suitable excipients may be optionally added to the formulation. Such excipients include glycine at suitable concentration so as to further stabilize the formulation.

The novel formulations of PEG-interferon alpha conjugates are prepared using suitable combinations of a buffer, stabilizer, cryoprotectant(s) &/or lyoprotectant(s) and a solvent, optionally with other excipients and suitably lyophilized and stored as a dry powder to be reconstituted before use.

The formulations prepared such, contain an effective amount of biologically active PEG-interferon alpha conjugates, and are useful in treating several diseases such as Hepatitis B & C and cancer etc. They are preferably used as injectable aqueous solutions.

Following non-limiting examples illustrate the described pharmaceutical compositions of the present invention and the means of carrying out the invention to obtain a stable pharmaceutical dosage form of PEG-interferon alpha conjugates. It will be appreciated that the Examples are illustrative and such other suitable modifications/additions etc. as are well within the scope of the persons skilled in the art are meant to be encompassed within the scope of the present invention.

### EXAMPLES

Various formulations of PEG-interferon alpha-2b conjugates dissolved in suitable buffer were prepared in the presence of suitable cryoprotectant(s) and ' stabilizer(s) from those described in the specification and subsequently lyophilized. After lyophilization, samples were stored at 5 °C (± 3 °C) and, at different periods of time, samples were reconstituted with water for analysis. The reconstituted samples were analyzed for visual clarity, *in-vitro* antiviral activity, and level of free interferon.

In an *in-vitro* antiviral assay, fresh, non-formulated, and formulated PEG-interferon alpha conjugate showed a specific activity in the range of 0.1 × 10⁸ to 0.8 × 10⁸ IU per milligram of interferon protein.

### Example 1

PEG-interferon alpha conjugate prepared as per techniques known in the art was formulated as described in Table 1.

**Table 1**

| Components of the Formulation of PEG-Interferon Alpha Conjugate Submitted to Lyophilization | |
|---|---|
| Components | Concentrations |
| PEG₁₂₀₀₀-interferon alpha-2b | 0.2 mg / mL* |
| Sodium succinate, pH 6.8 | 10 mM |
| Raffinose | 42.85 mg / mL |
| Polysorbate 80 | 0.1 mg / mL |
| Purified water | q.s. |

| | |
|---|---|
| * Based on protein weight | |

Lyophilization was carried out by placing the above specified solution in glass containers followed by loading the glass containers in to a lyophilizer at ambient pressure and temperature. Samples were frozen gradually in controlled manner at ambient pressure by lowering down the temperature up to -55 °C over a period of 11.5 h. Subsequently, frozen samples were subjected to primary drying in a step wise manner for 23 h, while raising the temperature from -55 °C to 0 °C and reducing the pressure from ambient to 50 mTorr. Following the primary drying cycles, secondary drying cycles were carried out for at least 16 h, while reducing the pressure from 50 to 20 mTorr. Upon completion of the lyophilization cycles, glass containers containing the lyophilized cakes were unloaded at ambient temperature and pressure.

After lyophilization, vials were collected and stored at 5 (±3) °C, until it was used for further analysis. Samples were reconstituted with water for analysis at different periods of time, as specified in Table 2. The reconstituted solutions were checked for visual clarity. Stability of PEG-interferon alpha conjugate was assessed for its *in-vitro* antiviral activity and level of free interferon (degree of depegylation) present in the reconstituted solution, as shown in Table 2.

| Table 2 | | | | |
|---|---|---|---|---|
| Stability of Formulated PEG-Interferon Alpha Conjugate After Lyophilization | | | | |
| Time (months) | Storage Temp. (± 3 °C) | Potency (IU / mg) | % Free IFN | Visual Clarity |
| Initial | | 0.38 × 10⁸ | 2.8 | CS |
| 3 | | 0.27 × 10⁸ | 2.8 | CS |
| 6 | 5 | 0.41 × 10⁸ | 3.6 | CS |
| 9 | | 0.45 × 10⁸ | 4.8 | CS |

| | | | | |
|---|---|---|---|---|
| CS - clear solution | | | | |

### Example 2

PEG-interferon alpha conjugate prepared as per techniques known in the art was formulated as described in Table 3.

**Table 3**

| Components of the Formulation of PEG-interferon Alpha Conjugate Submitted to Lyophilization | |
|---|---|
| Components | Concentrations |
| PEG₁₂₀₀₀-interferon alpha-2b | 0.2 mg / mL* |
| Sodium succinate, pH 6.8 | 10 mM |
| Lactose | 28.5 mg / mL |
| Raffinose | 42.85 mg / mL |
| Glycine | 1.05 mg / mL |
| Polysorbate 80 | 0.1 mg / mL |
| Purified water | q.s. |

| | |
|---|---|
| * Based on protein weight | |

Lyophilization was carried out in the same manner as described with Example 1.

After lyophilization, vials were collected and stored at 5 (±3) °C, until it was used for further analysis. Samples were reconstituted with water for analysis, at different periods of time, as specified in Table 4. The reconstituted solutions were checked for visual clarity. Stability of PEG-interferon alpha conjugate was assessed for its *in-vitro* antiviral activity and level of free interferon (degree of depegylation) present in the reconstituted solution, as shown in Table 4.

| Table 4 | | | | |
|---|---|---|---|---|
| Stability of Formulated PEG-Interferon Alpha Conjugate After Lyophilization | | | | |
| Time (months) | Storage Temp. (± 3°C) | Potency (IU / mg) | % Free. IFN | Visual Clarity |
| Initial | | 0.38 × 10⁸ | 2.94 | CS |
| 3 | | 0.18 × 10⁸ | 2.19 | CS |
| 6 | 5 | 0.46 × 10⁸ | 3.22 | CS |
| 9 | | 0.16 × 10⁸ | 4.0 | CS |

| | | | | |
|---|---|---|---|---|
| CS - clear solution | | | | |

### Example 3

PEG-interferon alpha conjugate prepared as per techniques known in the art was formulated as described in Table 5.

**Table 5**

| Components of the Formulation of PEG-Interferon Alpha Conjugate Submitted to Lyophilization | |
|---|---|
| Components | Concentrations |
| PEG₁₂₀₀₀-interferon alpha-2b | 0.2 mg / mL* |
| Sodium succinate, pH 6.8 | 10 mM |
| Lactose | 71.43 mg / mL |
| Raffinose | 21.43 mg / mL |
| Glycine | 1.05 mg / mL |
| Polysorbate 80 | 0.1 mg / mL |
| Purified water | q.s. |

| | |
|---|---|
| * Based on protein weight | |

Lyophilization was carried out in the same manner as described with Example 1.

After lyophilization, vials were collected and stored at 5 (±3) °C, until it was used for further analysis. Samples were reconstituted with water for analysis at different periods of time, as specified in Table 6. The reconstituted solutions were checked for visual clarity. Stability of PEG-interferon alpha conjugate was assessed for its *in-vitro* antiviral activity and level of free interferon (degree of depegylation) present in the reconstituted solution, as shown in Table 6.

| Table 6 | | | | |
|---|---|---|---|---|
| Stability of Formulated PEG-interferon Alpha Conjugate After Lyophilization | | | | |
| Time (months) | Storage Temp. (± 3 °C) | Potency (IU / mg) | % Free IFN | Visual Clarity |
| Initial | | 0.38 × 10⁸ | 2.8 | CS |
| 3 | | 0.14 × 10⁸ | 2.56 | CS |
| 6 | 5 | 0.42 × 10⁸ | 2.53 | CS |
| 9 | | 0.16 × 10⁸ | 3.52 | CS |

| | | | | |
|---|---|---|---|---|
| CS - clear solution | | | | |

### Example 4

PEG-interferon alpha conjugate prepared as per techniques known in the art was formulated as described in Table 7.

**Table 7**

| Components of the Formulation of PEG-Interferon Alpha Conjugate Submitted to Lyophilization | |
|---|---|
| Components | Concentrations |
| PEG₁₂₀₀₀-interferon alpha-2b | 0.2 mg / mL* |
| Sodium succinate, pH 6.8 | 10 mM |
| Raffinose | 57.14 mg / mL |
| Trehalose | 22.85 mg / mL |
| Glycine | 7.14 mg / mL |
| Polysorbate 80 | 0.1 mg / mL |
| Purified water | q.s. |

| | |
|---|---|
| * Based on protein weight | |

Lyophilization was carried out in the same manner as described with Example 1.

After lyophilization, vials were collected and stored at 5 (±3) °C, until it was used for further analysis. Samples were reconstituted with water for analysis at different periods of time, as specified in Table 8. The reconstituted solutions were checked for visual clarity. Stability of PEG-interferon alpha conjugate was assessed for its *in-vitro* antiviral activity and level of free interferon (degree of depegylation) present in the reconstituted solution, as shown in Table 8.

| Table 8 | | | | |
|---|---|---|---|---|
| Stability of Formulated PEG-interferon Alpha Conjugate After Lyophilization | | | | |
| Time (months) | Temp. (± 3°C | Potency IU / mg) | % Free | Visual Clarity |
| Initial | | 0.38 × 10⁸ | 2.85 | CS |
| 3 | | 0.21 × 10⁸ | 2.47 | CS |
| 6 | 5 | nd | 4.07 | CS |
| 9 | | 0.16 × 10⁸ | 6.19 | CS |

| | | | | |
|---|---|---|---|---|
| nd - not determined; CS - clear solution | | | | |

Free IFN content in all the examples above was determined by HP-size exclusion chromatography.

The novel lyophilized formulations of PEG-interferon alpha conjugates described in the present invention have the following advantages:
1. involve operational simplicity,
2. stabilize the PEG-IFN conjugates,
3. provide good physical strength to the lyophilized formulation,
4. reduce the lyophilization cycles, thereby, significantly reducing the cost and time of operation.

## Claims

1. A formulation comprising PEG-interferon alpha conjugate(s), buffer(s), stabilizer(s), cryoprotectant(s) and a solvent, wherein said cryoprotectant is raffinose.

2. A formulation as claimed in claim 1, wherein the stabilizer is selected from SDS or suitable polysorbates.

3. A formulation as claimed in claim 2, wherein the polysorbate is selected from polysorbate 20, 40 or 80.

4. A formulation as claimed in claim 1, wherein the buffer is selected from suitable succinate, phosphate, histidine or glycinate buffers either alone or in combination.

5. The formulation as claimed in claim 4, wherein the buffer is selected from sodium succinate, potassium succinate, sodium phosphate, histidine chloride, sodium glycinate either alone or in combination.

6. The formulation as claimed in any of the preceding claims, wherein the said buffer is sodium succinate.

7. The formulation of any of the preceding claims, wherein the pH of the said formulation is in the range of 4.0-7.0.

8. The formulation as claimed in any of preceding claims, wherein the cryoprotectant further comprises lactose or trehalose.

9. The formulation as claimed in any of the preceding claims, wherein the concentration of the said PEG-interferon alpha conjugates is 0.03 to 2.0 mg interferon alpha per mL.

10. The formulation as claimed in any of the preceding claims, wherein the said stabilizer is present in a concentration of 0.01 to 1.0 mg/mL.

11. The formulation as claimed in any of the preceding claims, wherein the concentration of buffer is in the range of 0.001 to 1.0 M.

12. The formulation as claimed in any of the preceding claims, wherein the concentration of cryoprotectant is between 10-100 mg / mL.

13. The formulation as claimed in any of the preceding claims, wherein said solvent is water.

14. The formulation as claimed in any of the preceding claims, wherein the composition further comprises glycine.

15. The formulation as claimed in claim 14, wherein the concentration of glycine is between 0.1-100 mg / mL.

16. The formulation as claimed in any of the preceding claims, wherein said PEG-interferon alpha conjugates comprise predominantly single PEG molecules conjugated to single interferon alpha molecules.

17. The formulation as claimed in any of the preceding claims, wherein said interferon alpha molecules are selected from the group consisting of interferon alpha-2a, interferon alpha-2b, interferon alpha-2c or their suitable variants.

18. The formulation as claimed in any of the preceding claims, wherein said interferon alpha molecules are interferon alpha-2b.

19. A formulation of any preceding claims comprising PEG-interferon alfa-2b, raffinose as the cryoprotectant, Tween 80 as the stabilizer, sodium succinate as the buffer and water as solvent.

20. A formulation as claimed in claim 19, wherein the cryoprotectant further comprises lactose or trehalose.

21. A process of lyophilizing the formulation as claimed in any of the preceding claims, to obtain a lyophilized powder.

22. A lyophilized formulation prepared according to the process as claimed in claim 21.

23. A formulation consisting of PEG-interferon alpha conjugate(s), buffer(s), stabilizer(s), cryoprotectant(s) and a solvent, wherein said cryoprotectant is raffinose.

24. The formulation as claimed in any preceding claim, wherein the lyophilized powder is reconstituted with water prior to administration.

25. A process of preparing a lyophilized formulation of PEG-interferon alfa conjugates comprising, combining said conjugates with a suitable buffer, a suitable stabilizer, a suitable cryoprotectant and a solvent, as claimed in any of the preceding claims, wherein the said cryoprotectant is raffinose and, subsequently, lyophilizing the mixture to obtain a lyophilized powder.

## Patentansprüche

1. Formulierung, umfassend PEG-Interferon-alpha-Konjugat(e), Puffer, Stabilisator(en), Gefrierschutzmittel bzw. Kryoprotektivum und ein Lösungsmittel, wobei das Gefrierschutzmittel Raffinose ist.

2. Formulierung gemäß Anspruch 1, wobei der Stabilisator aus SDS oder geeigneten Polysorbaten gewählt wird.

3. Formulierung gemäß Anspruch 2, wobei das Polysorbat aus Polysorbat 20, 40 oder 80 gewählt wird.

4. Formulierung gemäß Anspruch 1, wobei der Puffer aus geeigneten Succinat-, Phosphat-, Histidin- oder Glycinatpuffern entweder allein oder in Kombination gewählt wird.

5. Formulierung gemäß Anspruch 4, wobei der Puffer aus Natriumsuccinat, Kaliumsuccinat, Natrium-phosphat, Histidinchlorid, Natriumglycinat entweder allein oder in Kombination gewählt wird.

6. Formulierung gemäß einem beliebigen der vorhergehenden Ansprüche, wobei der Puffer Natriumsuccinat ist.

7. Formulierung gemäß einem beliebigen der vorhergehenden Ansprüche, wobei der pH-Wert der Formulierung im Bereich von 4,0 - 7,0 liegt.

8. Formulierung gemäß einem beliebigen der vorhergehenden Ansprüche, wobei das Gefrierschutzmittel weiterhin Lactose oder Trehalose umfasst.

9. Formulierung gemäß einem beliebigen der vorhergehenden Ansprüche, wobei die Konzentration der PEG-Interferon-alpha-Konjugate 0,03 bis 2,0 mg Interferon-alpha pro mL beträgt.

10. Formulierung gemäß einem beliebigen der vorhergehenden Ansprüche, wobei der Stabilisator in einer Konzentration von 0,01 bis 1,0 mg/mL vorliegt.

11. Formulierung gemäß einem beliebigen der vorhergehenden Ansprüche, wobei die Konzentration von Puffer im Bereich von 0,001 bis 0,5 M beträgt.

12. Formulierung gemäß einem beliebigen der vorhergehenden Ansprüche, wobei die Konzentration von Gefrierschutzmittel zwischen 10 - 100 mg/mL liegt.

13. Formulierung gemäß einem beliebigen der vorhergehenden Ansprüche, wobei das Lösungsmittel Wasser ist.

14. Formulierung gemäß einem beliebigen der vorhergehenden Ansprüche, wobei die Zusammensetzung weiterhin Glycin umfasst.

15. Formulierung gemäß Anspruch 14, wobei die Konzentration von Glycin zwischen 0,1 - 100 mg/mL liegt.

16. Formulierung gemäß einem beliebigen der vorhergehenden Ansprüche, wobei die PEG-Interferon-alpha-Konjugate vorwiegend einzelne PEG-Moleküle, die an einzelne Interferon-alpha-Moleküle konjugiert sind, umfassen.

17. Formulierung gemäß einem beliebigen der vorhergehenden Ansprüche, wobei die Interferon-alpha-Moleküle aus der Gruppe, die aus Interferon-alpha-2a, Interferon-alpha-2b, Interferon-alpha-2c oder ihren geeigneten Varianten besteht, gewählt werden.

18. Formulierung gemäß einem beliebigen der vorhergehenden Ansprüche, wobei die Interferon-alpha-Moleküle Interferon-alpha-2b sind.

19. Formulierung gemäß einem beliebigen der vorhergehenden Ansprüche, umfassend PEG-Interferon-alpha-2b, Raffinose als Gefrierschutzmittel, Tween 80 als Stabilisator, Natriumsuccinat als Puffer und Wasser als Lösungsmittel.

20. Formulierung gemäß Anspruch 19, wobei das Gefrierschutzmittel weiterhin Lactose oder Trehalose umfasst.

21. Verfahren zur Lyophilisierung der Formulierung gemäß einem beliebigen der vorhergehenden Ansprüche, um ein lyophilisiertes Pulver zu erhalten.

22. Lyophilisierte Formulierung, hergestellt gemäß dem Verfahren gemäß Anspruch 21.

23. Formulierung, bestehend aus PEG-Interferon-alpha-Konjugat(en), Puffer(n), Stabilisator(en), Gefrierschutzmittel(n) und einem Lösungsmittel, wobei das Gefrierschutzmittel Raffinose ist.

24. Formulierung gemäß einem beliebigen der vorhergehenden Ansprüche, wobei das lyophilisierte Pulver mit Wasser vor der Verabreichung rekonstituiert wird.

25. Verfahren zur Herstellung einer lyophilisierten Formulierung von PEG-Interferon-alpha-Konjugaten, umfassend das Kombinieren die konjugaten, eines geeigneten Puffers, eines geeigneten Stabilisators, eines geeigneten Gefrierschutzmittels und eines Lösungsmittels gemäß einem beliebigen der vorhergehenden Ansprüche, wobei das Gefrierschutzmittel Raffinose ist, und anschließend das Lyophilisieren der Mischung für den Erhalt eines lyophilisierten Pulvers.

## Revendications

1. Formulation comprenant un ou des conjugué(s) de polyéthylène-glycol et d'interféron alpha, un ou des agent(s) tampon(s), un ou des stabilisant(s), un ou des agent(s) cryoprotecteur(s), et un solvant, dans laquelle ledit agent cryoprotecteur est du raffinose.

2. Formulation conforme à la revendication 1, pour laquelle le stabilisant est choisi parmi le dodécane-sulfate de sodium (SDS) et les polysorbates appropriés.

3. Formulation conforme à la revendication 2, pour laquelle le polysorbate est choisi parmi les polysorbates 20, 40 et 80.

4. Formulation conforme à la revendication 1, pour laquelle l'agent tampon est choisi parmi les agents tampons de type succinate, phosphate, histidine ou glycinate appropriés, qui sont utilisés seuls ou en combinaison.

5. Formulation conforme à la revendication 4, pour laquelle l'agent tampon est choisi parmi du succinate de sodium, du succinate de potassium, du phosphate de sodium, du chlorure d'histidine et du glycinate de sodium, qui sont utilisés seuls ou en combinaison.

6. Formulation conforme à l'une des revendications précédentes, dans laquelle ledit agent tampon est du succinate de sodium.

7. Formulation conforme à l'une des revendications précédentes, laquelle formulation présente un pH valant de 4,0 à 7,0.

8. Formulation conforme à l'une des revendications précédentes, dans laquelle l'agent cryoprotecteur comprend en outre du lactose ou du tréhalose.

9. Formulation conforme à l'une des revendications précédentes, dans laquelle la concentration desdits conjugués de polyéthylèneglycol et d'interféron alpha équivaut à une concentration d'interféron alpha de 0,03 à 2,0 mg/mL.

10. Formulation conforme à l'une des revendications précédentes, dans laquelle ledit stabilisant se trouve présent en une concentration de 0,01 à 1,0 mg/mL.

11. Formulation conforme à l'une des revendications précédentes, dans laquelle la concentration de l'agent tampon vaut de 0,001 à 0,5 M.

12. Formulation conforme à l'une des revendications précédentes, dans laquelle la concentration de l'agent cryoprotecteur vaut de 10 à 100 mg/mL.

13. Formulation conforme à l'une des revendications précédentes, dans laquelle ledit solvant est de l'eau.

14. Formulation conforme à l'une des revendications précédentes, laquelle composition comprend en outre de la glycine.

15. Formulation conforme à la revendication 14, dans laquelle la glycine se trouve en une concentration de 0,1 à 100 mg/mL.

16. Formulation conforme à l'une des revendications précédentes, dans laquelle lesdits conjugués de polyéthylèneglycol et d'interféron alpha sont constitués en majorité d'une seule molécule de PEG conjuguée à une seule molécule d'interféron alpha.

17. Formulation conforme à l'une des revendications précédentes, pour laquelle les molécules d'interféron alpha sont choisies dans l'ensemble formé par l'interféron alpha-2a, l'interféron alpha-2b, l'interféron alpha-2c et leurs variantes appropriées.

18. Formulation conforme à l'une des revendications précédentes, dans laquelle les molécules d'interféron alpha sont des molécules d'interféron alpha-2b.

19. Formulation conforme à l'une des revendications précédentes qui comprend un conjugué de polyéthylèneglycol et d'interféron alpha-2b, du raffinose en qualité d'agent cryoprotecteur, du Tween 80 en qualité de stabilisant, du succinate de sodium en qualité d'agent tampon, et de l'eau comme solvant.

20. Formulation conforme à la revendication 19, dans laquelle l'agent cryoprotecteur comprend en outre du lactose ou du tréhalose.

21. Procédé de lyophilisation d'une formulation conforme à l'une des revendications précédentes, permettant d'obtenir une poudre lyophilisée.

22. Formulation lyophilisée, préparée selon un procédé conforme à la revendication 21.

23. Formulation constituée d'un ou de conjugué(s) de polyéthylène-glycol et d'interféron alpha, d'un agent ou d'agents tampon(s), d'un ou de stabilisant(s), d'un agent ou d'agents cryoprotecteur(s), et d'un solvant, dans laquelle ledit agent cryoprotecteur est du raffinose.

24. Formulation conforme à l'une des revendications 1 à 18 et 20 à 23, ladite poudre lyophilisée étant dissoute dans de l'eau pour reconstitution d'une solution, avant d'être administrée.

25. Procédé de préparation d'une formulation lyophilisée de conjugués de polyéthylène-glycol et d'interféron alpha, lequel procédé comporte le fait de combiner ces conjugués avec un agent tampon approprié, un stabilisant approprié, un agent cryoprotecteur approprié et un solvant, tels que mentionnés dans les revendications précédentes, ledit agent cryoprotecteur étant du raffinose, et le fait de lyophiliser ensuite le mélange ainsi préparé, de manière à obtenir une poudre lyophilisée.
